(19) Europäisches Patentamt
European Patent Office
Office européen des brevets

(11) Publication number: 0 178 917
A1

(12) # EUROPEAN PATENT APPLICATION

(21) Application number: 85307438.3

(22) Date of filing: 16.10.85

(51) Int. Cl.[4]: C 07 D 239/62
C 07 D 239/64

(30) Priority: 16.10.84 GB 8426101

(43) Date of publication of application: 23.04.86 Bulletin 86/17

(84) Designated Contracting States: BE CH DE FR GB IT LI NL SE

(71) Applicant: BP Chemicals Limited
Belgrave House 76 Buckingham Palace Road
London, SW1W 0SU(GB)

(72) Inventor: McKillop, Alexander
School of Chem. Sciences University of East Anglia
The Plain Norwich(GB)

(74) Representative: Fawcett, Richard Fennelly et al,
BP INTERNATIONAL LIMITED Patents Division Chertsey Road
Sunbury-on-Thames Middlesex, TW16 7LN(GB)

(54) **Process for the production of barbiturates.**

(57) Salts of barbituric acid are produced by reacting a urea with a diester of malonic acid at elevated temperature in the presence of a strong base which is an amidine.

EP 0 178 917 A1

PROCESS FOR THE PRODUCTION OF BARBITURATES

The present invention relates to the production of barbituric acid and salts of this acid known as barbiturates. In particular the invention relates to the production of these compounds by the reaction of a urea with a diester of a substituted or unsubstituted malonic acid in the presence of a base.

It is known that barbituric acid and its barbiturate salts can be prepared by reaction of urea with a malonic acid ester, and previously sodium alkoxides, for example sodium methoxide, have been used to effect the reaction. These alkoxides however have a number of disadvantages which can cause problems when the reaction is carried out commercially on a large scale. For example, the alkoxide salt poses considerable health and safety problems and is an air-sensitive solid and its storage and use on a large scale therefore requires special apparatus and precautions to be taken.

A second disadvantage associated with the use of sodium alkoxide is that it is not regenerable after the reaction has occurred. Thus, during the reaction, the sodium salt of barbituric acid is formed from which the sodium alkoxide is not regenerable. This necessitates using the alkoxide on a scale commensurate with amounts of product produced and furthermore, since the sodium salt of barbituric acid is only sparingly soluble in the reaction mixture, makes it difficult to operate the process continuously.

It has now been discovered, that strongly basic amidines can be used to effect the formation of barbituric acid salts. Such strong bases are air stable and easily regenerable and hence avoid many of

the problems associated with the use of sodium alkoxides.

Accordingly, the present invention provides a process for the production of salts of barbituric acid, which process comprises reacting a urea with a diester of a malonic acid at elevated temperature in the presence of a strong base characterised in that the strong base is an amidine.

Suitably the urea used as reactant is either urea itself, a N,N'-dialkyl or -diaryl urea or a thiourea. Preferred ureas include, urea, N,N'-diethylurea, N,N'-dimethylurea, N,N'-diphenylurea and its substituted derivatives.

As regards the diester of the malonic acid, this may be a diester of a substituted or unsubstituted malonic acid. Suitably the esters are dialkyl esters of malonic acid or a substituted malonic acid of formula $HO_2CC(R^1)(R^2)CO_2H$ where $R^1$ and $R^2$ are independently substituted, unsubstituted, saturated or unsaturated alkyl, cycloalkyl or aryl groups or hydrogen .

The product of the process described is the quaternised salt of barbituric acid, the quaternised cation being derived from the amidine catalyst. Preferred salts which can be formed by this reaction using the appropriate reactants are the salts of barbituric acid, 5,5-diethylbarbituric acid and 5-phenyl- 5-ethylbarbituric acid.

Preferably, the reaction is carried out in the presence of a solvent. If a solvent is used it is suitably an aliphatic alcohol, preferably methanol, ethanol or the isomeric propanols and butanols.

By the term amidine is meant a compound containing the grouping

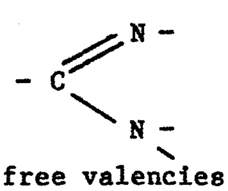

Conveniently the free valencies on the nitrogen atom are attached to carbon atoms or hydrogen and the free valency on the carbon to another carbon or nitrogen atoms. In the last mentioned case the structure will comprise a guanidine grouping.

A preferred class of amidines is the cyclic amidines. Cyclic amidines are defined as those amidines wherein at least one of the

nitrogen atoms is part of an alicyclic or heterocyclic substituted or unsubstituted hydrocarbyl ring. In the case where the amidine is a guanidine then any two of the three nitrogen atoms may be in the same or different rings. Those nitrogen atoms which are not part of any said ring may form part of a substituted or unsubstituted hydrocarbyl group.

A preferred class of cyclic amidine is that in which the amidine group can form part of a fused ring system containing 6 and 5 membered rings or 6 and 7 membered rings or two six membered rings, as for example 1,5-diazabicyclo[4.3.0]non-5-ene (DBN), or 1,8-diazabicyclo[5.4.0]undec-7-ene (DBU) or 1,5,7-triazabicyclo-[4.4.0]dec-5-ene (TBD).

The amidine may be added to the reaction mixture either on its own or as a solution in an alcohol solvent.

The amidine can be supported, that is chemically and physically bonded, to an inert solid and then added to the reaction mixture. In the supported form, the surface atoms of the solid are bonded to one or more of the free valencies of the amidine or guanidine group either directly or through an intermediate hydrocarbyl radical. In the case of cyclic amidines the hydrocarbyl radical may constitute part of the ring structure of the molecule.

The inert solid may be either organic, for example a polymer, copolymer, resin and the like or it may be inorganic such as a silica aluminosilicate, diatomaceous earth, zeolite or clay.

The amidine should be added in amounts corresponding to a molar ratio of the urea to amidine of 1:5 to 5:1.

As regards reaction conditions, it is preferred to carry out the reaction at elevated temperature and conveniently at the boiling point of the reaction mixture. The preferred range of reaction temperatures will usually depend on the exact reactants used but are typically in the range 50 to 150°C.

The reaction may be carried out at atmospheric pressure or, in the case where the reaction is carried out in a closed vessel, under the autogenous pressure of the reactants at the reaction temperature.

As regards the relative amounts of reactants it is preferable that the diester and the urea are fed in amounts corresponding to a molar ratio in the range 5:1 to 1:5.

Although the product of the above reaction is a quaternised salt of barbituric acid, the parent barbituric acid may be prepared by treating the product with acid, and the resulting amidine salt treated to recover the amidine for recycle and reuse. Accordingly, an embodiment of the present invention provides a process for the preparation of a barbituric acid from a urea and a diester of a malonic acid <u>characterised in that</u>

(a) in a first step, the diester of the malonic acid and the urea are reacted together at elevated temperature, in the presence of an amidine, to produce a salt of a barbituric acid, and

(b) in a second step, the salt of the barbituric acid or a reaction mixture containing the salt is treated with acid in order to produce the barbituric acid.

In a preferred third step (c) the amidine is recovered by any convenient means, for example using a basic ion exchange column which can subsequently be regenerated by treatment with dilute base.

The second stage may be effected using any acid catalyst but it is preferable to use a strong mineral acid e.g. hydrochloric acid.

The processes described may be carried out either batchwise or continuously.

The invention is illustrated by the following Examples.

<u>Example 1</u>

A 500 ml round bottomed flask fitted with a reflux condenser was charged with 17.4 g of 1,5,7-triazabicyclo[4.4.0]dec-5-ene (TBD), 20 g of diethyl malonate, 7.5 g of urea, and 125 ml of ethanol. The resulting solution was refluxed for 6 hours, cooled, and 100 ml of distilled water added followed by 11 ml of concentrated hydrochloric acid. The solution was cooled overnight in a refrigerator and the resulting crystals filtered, washed with cold water (25 ml) and dried in an oven at 100°C for 4 hours. Yield of barbituric acid = 7.2 g.

The mother liquor was passed through a 30 x 2 cm column of

IRA-93 ion-exchange resin (pre-washed with 1N NaOH). The resulting solution was evaporated to dryness to give an off white solid (17.8g). This solid was characterised as TBD by 'H.N.M.R.

Example 2

Example 1 was repeated except that 19 g of 1,8-diazabicyclo[5.4.0]unde-7-ene was used as a catalyst in place of TBD. Yield of barbituric acid = 3.2 g.

Example 3

Example 2 was repeated except that 125 ml of methanol was used in place of ethanol. Yield of barbituric acid = 4 g.

Claims:

1 A process for the production of salts of barbituric acid, which process comprises reacting a urea with a diester of a malonic acid at elevated temperature in the presence of a strong base characterised in that the strong base is an amidine.

2 A process according to claim 1 wherein the urea reactant is either urea itself, an N,N'-dialkyl urea, an N,N'-diaryl urea or a thiourea.

3 A process according to either claim 1 or claim 2 wherein the diester of malonic acid is either a dialkyl ester of malonic acid or a substituted malonic acid of the formula:

$$HO_2CC(R^1)(R^2)CO_2H$$

wherein $R^1$ and $R^2$ are independently substituted or unsubstituted, saturated or unsaturated alkyl, cycloalkyl or aryl groups or hydrogen.

4 A process according to any one of the preceding claims wherein the reaction is carried out in the presence of a solvent which is an aliphatic alcohol.

5 A process according to any one of the preceding claims wherein the amidine is a cyclic amidine.

6 A process according to claim 5 wherein the cyclic amidine is either 1,5-diazabicyclo[4.3.0]non-5-ene (DBN), 1,8-diazabicyclo[5.4.0]undec-7-ene (DBU) or 1,5,7-triazabicyclo[4.4.0]dec-5-ene (TBD).

**7** A process according to any one of claims 1 to 4 wherein the amidine is a guanidine.

**8** A process according to any one of the preceding claims wherein the catalyst is added in amounts corresponding to a molar ratio of the urea to catalyst of 1:5 to 5:1.

**9** A process according to any one of the preceding claims wherein the diester and the urea are fed in amounts corresponding to a molar ratio in the range 5:1 to 1:5.

**10** A process for the preparation of a barbituric acid from a urea and a diester of a malonic acid

characterised in that

(a) in a first step the diester of the malonic acid and the urea are reacted together at elevated temperature, in the presence of an amidine, to produce a salt of a barbituric acid, and

(b) in a second step the salt of the barbituric acid or a reaction mixture containing the salt is treated with acid in order to produce the barbituric acid.

**11** A process according to claim 10 wherein in a third step (c) the amidine is recovered.

**12** A process according to either claim 10 or claim 11 wherein the acid employed in step (b) is a strong mineral acid.

European Patent Office

# EUROPEAN SEARCH REPORT

0178917
Application number

EP 85 30 7438

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | CH-A- 167 802 (PARA)<br>* Pages 1,2 *<br>----- | 1-4 | C 07 D 239/62<br>C 07 D 239/64 |
| | | | TECHNICAL FIELDS SEARCHED (Int Cl 4) |
| | | | C 07 D 239/60 |

The present search report has been drawn up for all claims

| Place of search | Date of completion of the search | Examiner |
|---|---|---|
| THE HAGUE | 10-01-1986 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82